# EUROPEAN PATENT APPLICATION

(11) **EP 2 062 597 A1**
(43) Date of publication of application: **27.05.2009**
(21) Application number: 07828315.7
(22) Date of filing: 14.09.2007
(51) Int. Cl.: A61K 45/00, A61K 31/7088, A61K 39/395, A61K 48/00, A61P 35/00, A61P 43/00, C12Q 1/48, G01N 33/15, G01N 33/50, G01N 33/574

(54) **PREVENTIVE OR REMEDY FOR ER-NEGATIVE AND HER2-NEGATIVE BREAST CANCER AND METHOD OF SCREENING THE SAME**

(30) Priority: 15.09.2006 JP 2006250878
(71) Applicant: Tokai University, Tokyo 151-0063 (JP); Takeda Pharmaceutical Company Limited, Osaka-shi, Osaka 541-0045 (JP)
(72) Inventor: UMEMURA, Shinobu, Isehara-shi, Kanagawa 259-1143 (JP); TOKUDA, Yutaka, Isehara-shi, Kanagawa 259-1143 (JP); YOSHIDA, Sei, Tsukuba-shi, Ibaraki 300-4293 (JP); OTA, Yoshikazu, Tsukuba-shi, Ibaraki 300-4293 (JP)
(74) Representative: Jones, Nicholas Andrew
(86) International application number: PCT/JP2007/068482
(87) International publication number: WO 2008/032876

(57) **Abstract**

The present invention provides (1) an agent for the prevention or treatment of an estrogen receptor-negative and HER2-negative breast cancer comprising an Akt inhibitor, (2) an agent for the prevention or treatment of an estrogen receptor-negative, progesterone receptor-negative and HER2-negative breast cancer comprising an Akt inhibitor, (3) a method of screening an agent for the prevention or treatment of a breast cancer which is negative for hormone receptors such as an estrogen receptor, a progesterone receptor, etc. and is negative for HER2, which comprises using an Akt inhibitory activity as an indicator; and so on.

SEQUENCE LISTING

<110>  Takeda Pharmaceutical Company Limited

<120>  Agent for preventing or treating breast cancer with ER negative and HER2
negative, and method of screening the same

<130>  G08-0105US

<140>  PCT/JP2007/68482
<141>  2007-09-14

<150>  JP2006-250878
<151>  2006-09-15

<160>  2

<170>  PatentIn version 3.3

<210>  1
<211>  15
<212>  PRT
<213>  Artificial

<220>
<223>  Unknown

<400>  1

Ala Val Thr Asp His Pro Asp Arg Leu Trp Ala Trp Glu Lys Phe
1               5                   10                  15

<210>  2
<211>  26
<212>  PRT
<213>  Artificial

<220>
<223>  Unknown

<400> 2

Tyr Gly Arg Lys Lys Arg Arg Gln Arg Arg Arg Ala Val Thr Asp His
1               5                   10                  15
Pro Asp Arg Leu Trp Ala Trp Glu Lys Phe
            20                  25

## Description

### TECHNICAL FIELD

The present invention relates to an agent for the prevention or treatment of breast cancer, which comprises an Akt inhibitor, and more particularly to (1) an agent for the prevention or treatment of estrogen receptor (hereinafter also referred to as ER)-negative and HER2-negative breast cancer, (2) an agent for the prevention or treatment of ER-negative, progesterone receptor (hereinafter also referred to as PgR)-negative and HER2-negative breast cancer, and so on.

The present invention further relates to a method of screening an agent for the prevention or treatment of (1) ER-negative and HER2-negative breast cancer, (2) ER-negative, PgR-negative and HER2-negative breast cancer, etc., using an Akt inhibitory activity as an indicator.

### BACKGROUND ART

Women who suffer from breast cancer have been increasing each year. For women breast cancer currently has the highest frequency, overtaking stomach cancer. Even in Japan over 30,000 women are affected with the disease each year; breast cancer has ranked first in its age-adjusted morbidity rate among all female carcinomas and a little less than 10,000 dies of breast cancer.

Approximately 60 to 80% of breast cancer grows and spreads under the influence of female hormones (estrogen, progesterone), and hormone therapy (anti-estrogen drugs, etc.) is effective for breast cancer which has hormone receptors (ER, PgR) as indicators of these hormones.

HER2 (sometimes also called as erbB2) is a receptor having the activity of a tyrosine kinase that belongs to the EGF receptor family, and is a factor associated with the growth/malignant transformation of cancer. HER2 is overexpressed in various tumors including breast cancer, lung cancer, colorectal cancer, pancreatic cancer, ovarian cancer, etc. and the prognosis of patients expressing HER2 is poor (Seminar in Oncology, 27 (5), 2000, Supplement 9, 13-19). Thus, anti-HER2 therapy against HER2-expressing tumor is expected to suppress tumor growth and in fact, humanized monoclonal antibodies against HER2 (Herceptin, registered trademark) show clinical effects against chemotherapeutic agent-resistant metastatic breast cancer overexpressing HER2 (Non-Patent Document 1: Seminar in Oncology, 29 (3), Supplement 11, 2002, 38-43).

The following findings are reported on the relationship between intracellular signal transduction factor Akt also called as protein kinase B (PKB) and breast cancer:
i) phosphorylated Akt is positively correlated with poor prognosis in patients with breast cancer, metastasis or recurrence of breast cancer (Non-Patent Document 2: British Journal of Oncology, 86, S40-545, 2002);
ii) phosphorylated Akt is negatively correlated with hormone therapy sensitivity to breast cancer (Non-Patent Document 3: Oncogene 22, 3205-3212, 2003; Non-Patent Document 4: European Journal of Cancer, 42, 629-635, 2006);
iii) phosphorylated Akt is negatively correlated with radiotherapy sensitivity to breast cancer (Non-Patent Document 5: Molecular Cancer Therapy, 5(5), 1183-1189, 2006);
iv) phosphorylated Akt reduces drug sensitivity to anti-HER2 drugs for breast cancer (Non-Patent Document 6: Journal of Oncology, 23(11), 2469-2476, 2005; Non-Patent Document 7: Nature Clinical Practice: Oncology, 3(5), 269-280, 2006);
v) phosphorylated Akt (PI3K/PKB type) can be a good molecular target of therapeutic agents for breast cancer (Non-Patent Document 8: Breast Cancer, 13(2), 137-144, 2006).

### DISCLOSURE OF INVENTION

In recent years, drug therapy for breast cancer has made rapid progress but patients who have not yet expressed hormone receptors such as estrogen receptors (ER) or progesterone receptors (PgR) or HER2 receptors, or patients who, even though the expression of these receptors was confirmed at diagnosis, lost these receptors during drug therapy to become irresponsive to the therapy (inadaptability/resistance), does not enjoy the benefits of therapy. Under such circumstances, drugs and chemotherapy which are effective for double negative patients with breast cancer who are both ER-negative and HER2-negative and triple negative patients with breast cancer who are ER-negative, HER2-negative and PgR negative have been earnestly desired. Particularly in triple negative breast cancer, therapy has not yet been established and there is no effective drug, resulting in poor prognosis is poor. It has thus been strongly desired to develop its therapeutic drug.

The present inventors have conducted extensive studies to solve the foregoing problems and as a result, found that phosphorylation of Akt is increased in (1) ER-negative and HER2-negative breast cancer tissues and (2) ER-negative, PgR-negative and HER2-negative breast cancer tissues and that signaling pathways involving Akt are significantly associated with the growth and malignant transformation of breast cancer which is negative for hormone receptors such as ER, PgR, etc. or HER2 receptors. The present invention has thus been accomplished.

More specifically, the present invention provides an agent for the prevention or treatment of breast cancer comprising the Akt inhibitor shown below, a method of screening such an agent for the prevention or treatment of breast cancer, and so on.
[1] An agent for the prevention or treatment of an estrogen receptor-negative and HER2-negative breast cancer comprising an Akt inhibitor.
[2] An agent for the prevention or treatment of an estrogen receptor-negative, progesterone receptor-negative and HER2-negative breast cancer comprising an Akt inhibitor.
   [2a] An agent for the prevention or treatment of an estrogen receptor-negative, progesterone receptor-positive and HER2-negative breast cancer comprising an Akt inhibitor.
   [2b] An agent for the prevention or treatment of a progesterone receptor-negative, and HER2-negative breast cancer comprising an Akt inhibitor.
   [2c] An agent for the prevention or treatment of a progesterone receptor-negative, estrogen receptor-positive and HER2-negative breast cancer comprising an Akt inhibitor.
[3] The agent for the prevention or treatment of the breast cancer according to [1] to [2c] above, wherein Akt is at least one selected from Akt1, Akt2 and Akt3.
   [3 a] The agent for the prevention or treatment of a breast cancer according to any one of [1] to [3] above, wherein the Akt inhibitor is a substance inhibiting the phosphorylation of Akt or a substance inhibiting the expression of Akt.
[4] The agent for the prevention or treatment of the breast cancer according to [1] to [3] above, wherein the Akt inhibitor is at least one selected from the group consisting of (a) a low molecular weight compound or high molecular weight compound which inhibits the phosphorylation of Akt, (b) a low molecular weight compound or high molecular weight compound which inhibits the expression of Akt, (c) an antibody which inhibits the phosphorylation of Akt, (d) an antibody which inhibits the expression of Akt, (e) a siRNA or shRNA against a polynucleotide encoding Akt, (f) an antisense polynucleotide comprising a nucleotide sequence complementary or substantially complementary to the nucleotide sequence of a polynucleotide encoding Akt, or comprising a part of said nucleotide sequence, (g) a ribozyme directed to a polynucleotide encoding Akt, (h) a mutant of Akt which dominant-negatively acts on Akt or a polynucleotide encoding said mutant, and (i) an aptamer against Akt.
   [4a] The agent for the prevention or treatment of the breast cancer according to any one of [1] to [3] above, wherein the Akt inhibitor is at least one selected from 1L-6-hydroxymethyl-chiro-inositol 2-(R)-2-O-methyl-3-O-octadecylcarbonate, SH-5, SH-6, NL-71-101, a peptide having the amino acid sequence of SEQ ID NO: 1 (H-AVTDHPDRLWAWEKF-OH), a peptide having the amino acid sequence of SEQ ID NO: 2 (H-YGRKKRRQRRR-AVTDHPDRLWAWEKF-OH) and TAT-TCL110-24.
[5] A method of screening an agent for the prevention or treatment of an estrogen receptor-negative and HER2-negative breast cancer, which comprises using an Akt inhibitory activity as an indicator.
[6] A method of screening an agent for the prevention or treatment of an estrogen receptor-negative, progesterone receptor-negative and HER2-negative breast cancer, which comprises using an Akt inhibitory activity as an indicator.
   [6a] A method of screening an agent for the prevention or treatment of a hormone receptor-negative (ER-negative and/or PgR-negative) and HER2-negative breast cancer, which comprises comparing phosphorylated Akt levels in breast cancer cells between (i) the case wherein a test compound is contacted with a hormone receptor-negative (ER-negative and/or PgR-negative) and HER2-negative breast cancer cells and (ii) the case wherein a test compound is not contacted with said cells.
[7] A method of screening an agent for the prevention or treatment of an estrogen receptor-negative, progesterone receptor-positive and HER2-negative breast cancer, which comprises using an Akt inhibitory activity as an indicator.
[8] A method of screening an agent for the prevention or treatment of a progesterone receptor-negative and HER2-negative breast cancer, which comprises using an Akt inhibitory activity as an indicator.
[9] A method of screening an agent for the prevention or treatment of a progesterone receptor- negative, estrogen receptor-positive and HER2-negative breast cancer, which comprises using an Akt inhibitory activity as an indicator.
[10] The agent for the prevention or treatment of a breast cancer according to any one of [1] to [4] above, wherein the negativity of estrogen receptor is defined as less than 10% in terms of a rate of positive cells with stained nucleus to the entire tumor when the estrogen receptor protein present in a breast cancer tissue section is detected by immunohistochemical staining.
[11] The agent for the prevention or treatment of a breast cancer according to any one of [1] to [4] above, wherein the negativity of progesterone receptor is defined as less than 10% in terms of a rate of positive cells with stained nucleus to the entire tumor when the progesterone receptor protein present in a breast cancer tissue section is detected by immunohistochemical staining.
[12] The agent for the prevention or treatment of a breast cancer according to any one of [1] to [4] above, wherein the negativity of HER2 is defined as less than 10% in terms of a rate of positive cells wherein the whole cell membrane is stained from weak to moderate intensity to the entire tumor when the HER2 protein present in a breast cancer tissue section is detected by immunohistochemical staining.
[13] Use of an Akt inhibitor for the prevention or treatment of an estrogen receptor-negative and HER2-negative breast cancer.
[14] Use of an Akt inhibitor for the prevention or treatment of an estrogen receptor-negative, progesterone-negative and HER2-negative breast cancer.
[15] Use of an Akt inhibitor to produce an agent for the prevention or treatment of an estrogen receptor-negative and HER2-negative breast cancer.
[16] Use of an Akt inhibitor to produce an agent for the prevention or treatment of an estrogen receptor-negative, progesterone-negative and HER2-negative breast cancer.
[17] A method of preventing or treating an estrogen receptor-negative and HER2-negative breast cancer, which comprises administering an effective does of an Akt inhibitor to a patient necessary to prevent or treat the breast cancer.
[18] A method of preventing or treating an estrogen receptor-negative, progesterone-negative and HER2-negative breast cancer, which comprises administering an effective does of an Akt inhibitor to a patient necessary to prevent or treat the breast cancer.

According to the present invention, there can be provided an agent for the prevention or treatment, which is effective for (1) an estrogen receptor-negative and HER2-negative breast cancer, (2) a progesterone-negative and HER2-negative breast cancer, (3) an estrogen receptor-negative, progesterone-negative and HER2-negative breast cancer, (4) an estrogen receptor-negative, progesterone-positive and HER2-negative breast cancer, (5) an estrogen receptor-positive, progesterone-negative and HER2-negative breast cancer, etc.

The present invention can further provide a drug and chemotherapy effective also for breast cancer patients who have not expressed hormone receptors such as ER, PgR, etc. and HER2 receptors for congenital or acquired reasons, or also for breast cancer patients who, even though the expression of these receptors was confirmed at diagnosis, lost these receptors during drug therapy to become irresponsive to the therapy (inadaptability/resistance).

Further according to the present invention, there can be provided a method of screening a drug effective for the treatment of a breast cancer in which hormone receptors and HER2 receptors have not been expressed, using Akt phosphorylation as an indicator.

### BRIEF DESCRIPTION OF DRAWINGS

FIG 1 presents the mean values for ratios of the protein levels to phosphorylated protein levels of HER2, Akt and MAPK in each group of the A-ER, B-ER, C-ER and D-ER groups classified by dividing samples according to the category (c).
FIG. 2 presents the mean values for ratios of the protein levels to phosphorylated protein levels of HER2, Akt and MAPK in each group of the A-PgR, B-PgR, C-PgR and D-PgR groups classified by dividing samples according to the category (c).
FIG 3 presents the mean values for ratios of the protein levels to phosphorylated protein levels of HER2, Akt and MAPK in Group (vi) (ER-negative, PgR-negative and HER2-negative groups) and the other groups (Groups (i) through (v)) classified by dividing samples according to the category (d).

### BEST MODES FOR CARRYING OUT THE INVENTION

### 1. Agent for the prevention or treatment of breast cancer comprising an Akt inhibitor.

First, the present invention provides the agent for the prevention or treatment of a breast cancer comprising the Akt inhibitor which is an agent for the prevention or treatment of (1) an estrogen receptor (ER)-negative and HER2-negative breast cancer; (2) an ER-negative, progesterone receptor (PgR)-negative and HER2-negative breast cancer, etc.

### 1.1 Akt inhibitor used in the present invention

"Akt" referred to as a v-akt murine thymoma viral oncogene homolog is an intracellular signaling factor, which is also termed as protein kinase B (PKB). Akt is a kind of serine/threonine kinase, reportedly takes an important role in the process of cancer progression, the regulation of insulin signaling and glucose metabolism and the neuron functions, and is thus the target for anticancer drugs, antidiabetic drugs and therapeutic drugs for cerebral infarction and neurodegeneration. Akt is known so far to exist in three isoforms of Akt1, Akt2 and Akt3.

"Akt1" is registered under Accession No. Gene: NM_001014432, Protein: NP_001014432 (Staal, S.P., Molecular cloning of the akt oncogene and its human homologues AKT1 and AKT2: amplification of AKT1 in a primary human gastric adenocarcinoma, Proc. Natl. Acad. Sci. U.S.A., 84 (14), 5034-5037 (1987)).

"Akt2" is registered under Accession No. Gene: NM_001626, Protein: NP_001617 (Staal, S.P., Molecular cloning of the akt oncogene and its human homologues AKT1 and AKT2: amplification of AKT1 in a primary human gastric adenocarcinoma, Proc. Natl. Acad. Sci. U.S.A., 84 (14), 5034-5037 (1987)).

"Akt3" is registered under Accession No. Gene: NM_005465, Protein: NP_005456 (Li, W., Zhang, J., Bottaro, D.P. and Pierce, J.H., Identification of serine 643 of protein kinase C-delta as an important autophosphorylation site for its enzymatic activity, J. Biol. Chem. 272 (39), 24550-24555 (1997)).

As used herein, "Akt" generally refers to an Akt protein, and sometimes refers to an Akt gene.

The "Akt inhibitor" refers to a substance that inhibits the phosphorylation of Akt and means a substance that inhibits the signal transduction mediated by Akt. The inhibition includes both complete interruption and weakening of signals. Such substances which can be used include not only a low molecular weight or high molecular weight compound but also siRNA, shRNA, an antibody, an antisense, a peptide, a protein, an enzyme, etc.

The term "inhibit the phosphorylation of Akt" is intended to mean inhibiting the activation of Akt by inhibiting interaction with a protein that phosphorylates Akt, inhibiting the phosphorylation through alteration of phosphorylated sites of Akt not to be phosphorylated, or the like.

The term "inhibit the expression of Akt" is intended to mean inhibiting the production of said protein by inhibiting any one of a series of events up to production of the protein from a gene encoding the protein (which includes, e.g., transcription (mRNA production), translation (protein production)), including the inhibition of expression of a gene for the protein.

As the Akt inhibitor used in the present invention, the substance that inhibits the phosphorylation of Akt, the substance that inhibits the expression of Akt, etc. are used. Specifically,
(a) a low molecular weight or high molecular weight compound that inhibits the phosphorylation of Akt,
(b) a low molecular weight or high molecular weight compound that inhibits the expression of Akt,
(c) an antibody that inhibits the phosphorylation of Akt,
(d) an antibody that inhibits the expression of Akt,
(e) siRNA or shRNA for a polynucleotide encoding Akt,
(f) an antisense polynucleotide comprising the entire or part of a nucleotide sequence complementary or substantially complementary to a nucleotide sequence of a polynucleotide encoding Akt,
(g) a ribozyme to a polynucleotide encoding Akt
(h) a mutant of Akt acting dominant-negatively against Akt or a polynucleotide encoding the same, and
(i) a substance selected from the group consisting of aptamer to Akt, etc.

The term "low molecular weight substance" is used to mean an organic or inorganic substance having a molecular weight of 10, 000 or less (preferably, a molecular weight of 5, 000 or less, more preferably, a molecular weight of 2, 000 or less, most preferably, a molecular weight of 700 or less). Also, the term "high molecular weight substance" is used to mean an organic substance having a molecular weight of 10, 000 or more (preferably, a molecular weight of 50, 000 or more, more preferably, a molecular weight of 100, 000 or more).

As used herein, the term Akt is intended to further include its variants as long as they have substantially the same activities as those of Akt. The variants of the proteins described above include, for example, proteins having amino acid sequences, wherein 1 or at least 2 (e.g., approximately 1 to 30, preferably approximately 1 to 10, more preferably several (1 to 6)) amino acids are deleted, substituted, added and/or inserted in the amino acid sequences described in the literatures *supra*. Where insertion, deletion or substitution occurs in an amino acid sequence, positions for the insertion, deletion or substitution are not particularly limited.

As the activity of substantially the same nature, there are, for example, an intracellular signaling activity, and the like. The term "substantially the same nature" is used to mean that the nature of these activities is equivalent in terms of quality (e.g., physiologically or pharmacologically). It is thus preferred that fatty acid synthase activities, etc. are equivalent (e.g., approximately 0.01 to 100 times, preferably approximately 0.1 to 10 times, more preferably 0.5 to 2 times), but differences in quantitative factors such as a level of these activities, or such as a molecular weight of the protein may be present and allowable.

A partial peptide of Akt may be any partial peptide so long as it possesses properties similar to those of Akt described above.

For example, in the constituent amino acid sequence for the protein used in the present invention, peptides containing at least 20, preferably at least 50, more preferably at least 70, much more preferably at least 100 and most preferably at least 200 amino acids, can be used.

The partial peptide used in the present invention may be peptides wherein 1 or at least 2 (preferably approximately 1 to 20, more preferably approximately 1 to 10 and most preferably several (1 to 5)) amino acids may be deleted in its constituent amino acid sequence; peptides wherein 1 or at least 2 (preferably approximately 1 to 20, more preferably approximately 1 to 10 and most preferably several (1 to 6)) amino acids may be added in the amino acid sequence; peptides wherein 1 or at least 2 (preferably approximately 1 to 20, more preferably approximately 1 to 10 and most preferably several (1 to 6)) amino acids may be inserted in the amino acid sequence; or peptides wherein 1 or at least 2 (preferably approximately 1 to 20, more preferably approximately 1 to 10 and most preferably several (1 to 6)) amino acids may be substituted by other amino acids in the amino acid sequence.

The partial peptide used in the present invention or its salts can be prepared by publicly known methods for peptide synthesis, or the partial peptide can be prepared by cleaving the protein used in the present invention with an appropriate peptidase. For the methods for peptide synthesis, for example, either solid phase synthesis or liquid phase synthesis may be used. That is, the partial peptide or amino acids that can constitute partial peptide used in the present invention are condensed with the remaining part. Where the product contains protecting groups, these protecting groups are removed to give the desired peptide. Publicly known methods for condensation and elimination of the protecting groups are described in (i) - (v) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima, ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method; conversely when the partial peptide is obtained in a salt form, it can be converted into its free form or other salts by publicly known methods or its modifications.

The enzyme inhibitory activity of Akt can be measured using HTScan^{™} Akt1 Kinase Assay Kit (Cell Signaling Technology, Inc., catalog No. 7501), HTScan^{™} Akt2 Kinase Assay Kit (Cell Signaling Technology, Inc., catalog No. 7504), HTScan^{™} Akt3 Kinase Assay Kit (Cell Signaling Technology, Inc., catalog No. 7507), etc.

The phosphorylation level of Akt can be measured using, for example, CASE Kit for ACT (Ser 473) (trademark; COSMO BIO CO., LTD)

### (a) Low molecular weight compound or high molecular weight compound that inhibits the phosphorylation of Akt

The compound that inhibits the phosphorylation of Akt (including a compound in its salt form) can inhibit the activity of Akt, and is thus preferably used as a substance that inhibits the activity ofAkt. The compound used in the present invention that inhibits the phosphorylation ofAkt is not particularly limited so far as the compound can inhibit the phosphorylation, and includes, for example, a compound or its salts and the like that bind to Akt to inhibit the activity.

Examples of these compounds may be those selected from peptides, proteins, non-peptide compounds, synthetic compounds, fermentation products, cell extracts, plant extracts, animal tissue extracts, plasma, etc. These compounds may be novel or publicly known compounds. As salts of the compound, there are, for example, physiologically acceptable metal salts, ammonium salts, salts with organic bases, salts with inorganic acids, salts with organic acids, salts with basic or acidic amino acids, etc. Preferred examples of the metal salts include alkali metal salts such as sodium salts, potassium salts, etc.; alkaline earth meal salts such as calcium salts, magnesium salts, barium salts, etc.; aluminum salts, etc. Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Preferred examples of the salts with inorganic acids include salts with hydrochloric acid, hydrobromic acid, nitric acid, sulfuric acid, phosphoric acid, etc. Preferred examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferred examples of the salts with basic amino acids include salts with arginine, lysine, ornithine, etc., and preferred examples of the salts with acidic amino acids include salts with aspartic acid, glutamic acid, etc.

Among others, physiologically acceptable salts are preferred. For example, where the compounds contain acidic functional groups therein, examples include inorganic salts such as alkali metal salts (e.g., sodium salts, potassium salts, etc.), alkaline earth metal salts (e.g., calcium salts, magnesium salts, barium salts, etc.), ammonium salts, etc., and in the case that the compounds contain basic functional groups therein, examples include salts with inorganic acids such as hydrobromic acid, nitric acid, sulfuric acid, phosphoric acid, etc., salts with organic acids such as acetic acid, phthalic acid, fumaric acid, oxalic acid, tartaric acid, maleic acid, citric acid, succinic acid, methanesulfonic acid, p-toluenesulfonic acid, etc.

Examples of these compounds are Akt inhibitors, etc. Also, the compounds can be obtained by the screening methods which will be later described.

Specific examples of the Akt inhibitor include 1L-6-hydroxymethyl-chiro-inositol 2-(R)-2-O-methyl-3-O-octadecylcarbonate (Hu, Y., et al., 2000, J. Med. Chem., 43, 3045.), SH-5, SH-6 (Kozikowski, A.P., et al., 2003, J. Am. Chem. Soc., 125, 1144.), NL-71-101 (Reuveni, H., et al., 2002, Biochemistry, 41, 10304.), a peptide having the amino acid sequence of SEQ ID NO: 1 (H-AVTDHPDRLWAWEKF-OH), a peptide having the amino acid sequence of SEQ ID NO: 2 (H-YGRKKRRQRRR-AVTDHPDRLWAWEKF-OH), TAT-TCL110-24 (Hiromura, M., et al., 2004, J. Biol. Chem., 279, 53407.), etc. These Akt inhibitors can be used solely or in combination of two or more, if necessary.

### (b) Low molecular weight compound or high molecular weight compound that inhibits the expression of Akt

The compound that inhibits the expression of Akt (including a compound in its salt form) can inhibit the expression of Akt, and is thus preferably used as the substance that inhibits the expression of Akt. The compound that inhibits the expression of Akt is not particularly limited so far as the compound can inhibit the expression of Akt, and includes, for example, (i) a compound that inhibits the transcription from an Akt-encoding gene (DNA) into an Akt-encoding mRNA, (ii) a compound that inhibits the translation from an Akt-encoding mRNA into Akt, etc. (i) The compound that inhibits the transcription from an Akt-encoding gene (DNA) into an Akt-encoding mRNA is not particularly limited so far as the compound inhibits the transcription from an Akt-encoding gene (DNA) into an mRNA and includes, for example, a compound that binds to a factor associated with the transcription from an Akt-encoding gene (DNA) into an mRNA to inhibit the transcription, etc. (ii) The compound that inhibits the translation from an Akt-encoding mRNA to Akt is not particularly limited so far as the compound inhibits the translation from an Akt-encoding mRNA into Akt and includes, for example, a compound that binds to a factor associated with the translation from an Akt-encoding mRNA to Akt to inhibit the translation, etc.

Examples of these compounds are the Akt inhibitors described below, etc. Also, these compounds can be obtained by the screening methods which will be later described.

### (c) Antibody that inhibits the phosphorylation of Akt and (d) antibody that inhibits the expression of Akt

In the present invention, the purpose is to inhibit the phosphorylation of Akt, and any antibody can be used so long as it inhibits the phosphorylation of Akt. The antibody includes (1) an antibody capable of recognizing Akt or its partial peptide and inhibiting the phosphorylation of Akt, (2) an antibody capable of recognizing a protein that phosphorylates Akt and inhibiting the phosphorylation of Akt, and the like. These antibodies may be either polyclonal antibodies or monoclonal antibodies.

The antibodies against Akt or its partial peptide and the protein that phosphorylates Akt or its partial peptide, which are used in the present invention (hereinafter, sometimes merely referred to as the protein used in the present invention in the description of the antibodies) can be produced by a publicly known method of producing an antibody or antiserum, using as an antigen the protein used in the present invention.

### [Preparation of monoclonal antibody]

### (i) Preparation of monoclonal antibody-producing cells

The protein used in the present invention is administered to warm-blooded animals either solely or together with carriers or diluents to the site where antibody production is possible by the administration. In order to potentiate the antibody productivity upon administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mouse, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with a protein as an antigen directly or together with a carrier, adding an anti-immunoglobulin antibody (where mouse cells are used for the cell fusion, anti-mouse immunoglobulin antibody is used) labeled with a radioactive compound or an enzyme or Protein A and detecting the monoclonal antibody bound to the solid phase, and a method which comprises adding the supernatant of hybridoma to a solid phase adsorbed with an anti-immunoglobulin antibody or Protein A, adding a protein labeled with a radioactive compound or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (ii) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody].

### [Preparation of polyclonal antibody]

The polyclonal antibody of the present invention can be manufactured by publicly known methods or modifications thereof. For example, a warm-blooded animal is immunized with an immunogen (protein antigen) per se, or a complex of immunogen and a carrier protein is formed and the animal is immunized with the complex in a manner similar to the method described above for the manufacture of monoclonal antibodies. The product containing the antibody against the protein used in the present invention is collected from the immunized animal followed by separation and purification of the antibody.

In the conjugate of immunogen and a carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out according to the method for the separation and purification of immunoglobulins performed as in the separation and purification of monoclonal antibodies described above.

### (e) siRNA or shRNA to polynucleotide encoding Akt

The double-stranded RNA having RNAi action on the polynucleotide encoding Akt (e.g., siRNA or shRNA, etc. to the polynucleotide encoding Akt) is low toxic and can suppress the translation of a gene encoding Akt to downregulate the expression of Akt, and can thus be used advantageously as the compound that inhibit the expression of Akt. The double-stranded RNA having RNAi action on the polynucleotide encoding Akt as described above includes a double-stranded RNA containing a part of RNA encoding Akt (e.g., a siRNA (small (short) interfering RNA), a shRNA (small (short) hairpin RNA), etc.), and the like.

These double stranded RNAs can be manufactured by designing the same based on the sequence of the polynucleotide of the present invention, by modifications of publicly known methods (e.g., Nature, 411, 494, 2001; Japanese National Publication (Tokuhyo) No. 2002-516062; U.S. Patent Application No. 2002/086356; Nature Genetics, 24, 180-183, 2000; Genesis, 26, 240-244, 2000; Nature, 407, 319-320, 2002; Genes & Dev., 16, 948-958, 2002; Proc. Natl. Acad. Sci. USA., 99, 5515-5520, 2002; Science, 296, 550-553, 2002; Proc. Natl. Acad. Sci. USA, 99, 6047-6052, 2002; Nature Biotechnology, 20, 497-500, 2002; Nature Biotechnology, 20, 500-505, 2002; and Nucleic Acids Res., 30, e46, 2002).

The length of double stranded RNA having the RNAi action used in the present invention is usually 17 to 30 nucleotides, preferably 19 to 27 nucleotides, and more preferably 20 to 22 nucleotides.

### (f) Antisense polynucleotide having a complementary or substantially complementary nucleotide sequence to the nucleotide sequence of the polynucleotide encoding Akt

The antisense polynucleotide having a complementary or substantially complementary nucleotide sequence to the nucleotide sequence of the polynucleotide encoding Akt or partial peptide (preferably, DNA) (hereinafter these DNAs are sometimes simply referred to as the DNA used in the present invention in the description of antisense polynucleotide) can be any antisense polynucleotide, so long as it possesses the entire or a part of the nucleotide sequence complementary or substantially complementary to the nucleotide sequence of the DNA used in the present invention and is capable of suppressing the expression of said DNA, but preferred is antisense DNA.

The nucleotide sequence substantially complementary to the DNA as used in the present invention includes, for example, a nucleotide sequence having at least about 70% homology, preferably at least about 80% homology, more preferably at least about 90% homology and most preferably at least about 95% homology, to the full-length nucleotide sequence or to its partial nucleotide sequence of a nucleotide sequence complementary to the DNA used in the present invention (i.e., complementary strand to the DNA used in the present invention), and the like. Especially in the full-length nucleotide sequence of the complementary strand to the DNA used in the present invention, preferred are (i) an antisense polynucleotide having at least about 70% homology, preferably at least about 80% homology, more preferably at least about 90% homology and most preferably at least about 95% homology, to the complementary strand of the nucleotide sequence which encodes the N-terminal region of Akt (e.g., the nucleotide sequence around the initiation codon) in the case of antisense polynucleotide directed to translation inhibition and (ii) an antisense polynucleotide having at least about 70% homology, preferably at least about 80% homology, more preferably at least about 90% homology and most preferably at least about 95% homology, to the complementary strand of the full-length nucleotide sequence of the DNA used in the present invention having intron, in the case of antisense polynucleotide directed to RNA degradation by RNaseH, respectively.

The antisense polynucleotide is generally composed of nucleotides of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The nucleotide part (pyrimidine, purine) may also be chemically modified and may be any one which hybridizes to a DNA containing the nucleotide sequence represented by SEQ ID NO: 2, etc. These antisense polynucleotides may be synthesized using a publicly known DNA synthesizer, etc.

The antisense polynucleotide of the present invention may contain altered or modified sugars, bases or linkages, may be provided in a specialized form such as liposomes, microspheres, or may be applied to gene therapy, or may be provided in combination with attached moieties. Such attached moieties used include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol, and the like.

The inhibitory activity of the antisense polynucleotide can be investigated using the transformant of the present invention, the *in vivo* or *in vitro* gene expression system of the present invention, or the *in vivo* or *in vitro* translation system of Akt.

### (g) Ribozyme to the polynucleotide encoding Akt

The polynucleotide having a ribozyme activity against the polynucleotide encoding Akt can downregulate the expression of Akt, and is thus preferably used as the compound that inhibits the expression of Akt. These ribozyme can be manufactured by designing the same based on the sequence of the polynucleotide of the present invention, by modifications of publicly known methods (e.g., TRENDS in Molecular Medicine, 7, 221, 200; FEBS Lett., 228, 228, 1988; FEBS Lett., 239, 285, 1988; Nucl. Acids. Res., 17, 7059, 1989; Nature, 323, 349, 1986; Nucl. Acids. Res., 19, 6751, 1991; Protein Eng., 3, 733, 1990; Nucl. Acids Res., 19, 3875, 1991; Nucl. Acids Res., 19, 5125, 1991; Biochem. Biophys. Res. Commun., 186, 1271, 1992, etc.). For example, the ribozyme can be manufactured by ligating a publicly known ribozyme to a part of the RNA encoding Akt. The part of the RNA encoding Akt includes a contiguous part (RNA fragment) to the cleavage site on the RNA of the present invention, which can be cleaved by a publicly known ribozyme. The ribozymes described above include large ribozymes such as the group I intron-type or the M1 RNA contained in RNaseP, small ribozymes such as the hammerhead-type or the hairpin-type, etc. (Protein, Nucleic acid, and Enzyme, 35, 2191, 1990). For the hammerhead-type ribozymes, reference can be made on, e.g., FEBS Lett., 228, 228, 1988; FEBS Lett., 239, 285, 1988; Protein, Nucleic Acid, Enzyme, 35, 2191, 1990; Nucl. Acids Res., 17, 7059, 1989, etc. For the hairpin-type ribozymes, reference can be made on, e.g., Nature, 323, 349, 1986; Nucl. Acids Res., 19, 6751, 1991; KAGAKU-TO-SEIBUTSU, 30, 112, 1992; etc.

### (h) Variant of Akt acting dominant negatively against Akt or the polynucleotide encoding the same

The variant of Akt acting dominant negatively against Akt or the polynucleotide encoding the same can inhibit the activity of Akt, and hence can be preferably used as the compound that inhibits the activity of Akt. As used herein, the term "variant of the protein acting dominant negatively against Akt " means a protein having an action to inhibit (eliminate or diminish) the activity of Akt through expression of the variant (cf., IDENSHI-NO-KINO SOGAI JIKKEN-HO (Experimental Technique for Inhibiting Gene Function) edited by Kazunari Taira, YODOSHA Publishing Co., 26-32, 2001., etc.).

### (i) Aptamer to Akt

The aptamer to Akt can inhibit the activity or function of Akt, and is thus preferably used as the compound that inhibits the activity of Akt. The aptamer is obtained by publicly known methods, for example, SELEX (systematic evolution of ligands by exponential enrichment) (Annual Review of Medicine, 56, 555-583, 2005). Structure of the aptamer can be determined using publicly known methods, and the aptamer is produced in accordance with methods publicly known, based on the structure determined.

### 1.2 Target disease of the present invention

In the present invention, a medicament comprising the Akt inhibitor described above is used as the agent for the prevention or treatment of breast cancer.

Target disease of the agent for the prevention or treatment of the present invention includes (1) an estrogen receptor-negative and HER2-negative breast cancer, (2)a progesterone receptor-negative and HER2-negative breast cancer, (3) an estrogen receptor-negative, progesterone receptor-negative and HER2-negative breast cancer, (4) an estrogen receptor-negative, progesterone receptor-positive and HER2-negative breast cancer, (5) an estrogen receptor-positive, progesterone receptor-negative and HER2-negative breast cancer. Specific target diseases of the present invention include (1) an estrogen receptor-negative and HER2-negative breast cancer, and (3) an estrogen receptor-negative, progesterone receptor-negative and HER2-negative breast cancer. That is, the present invention provides the agent for the prevention or treatment of these breast cancers.

Hereinafter, the assessment tests for the negative/positive status of estrogen receptors, progesterone receptors and HER2 will be described.

### 1.2.1 Assessment test for the negative/positive status of HER2

"HER2 (human epithelial growth factor receptor type 2, avian erythroblastosis oncogene B 2)" is a cancer gene similar to human epidermal growth factor receptor gene, and sometimes also called neu, c-erbB2, etc. HER2 is a receptor-type tyrosine kinase and reportedly is overexpressed in 10-30% of the patients with breast cancer. It is revealed that HER2 transduces growth signal into a cell by its enzyme activity. Accordingly, it is said that HER2-overexpressing breast cancer has a high growth ability and metastatic ability and as a result, takes phenotype in advanced breast cancer such as chemoresistance, resistance to radiation therapy, poor prognosis, etc. Thus, anti-HER2 therapy against HER2-expressing tumor is expected to suppress tumor growth and humanized monoclonal antibodies against HER2 (Herceptin, registered trademark) exhibit clinical effects against chemotherapeutic agent-resistant metastatic breast cancer overexpressing HER2.

On the other hand, it is known that there are patients who have not expressed HER2 or patients who have lost HER2 during anti-HER2 therapy to become irresponsive to the therapy (inadaptability/resistance), and therapeutic drugs for HER2 receptor-negative breast cancer have been earnestly desired.

The present invention responds to these requirements and provides the agent for the prevention or treatment of at least HER2-negative breast cancer.

HER2 is registered under Accession No. Gene: NM_004448.2 Protein: NP_004439. The following documents are known for articles on cloning.
(1) King, C.R., Kraus, M.H. and Aaronson, S.A, Amplification of a novel v-erbB-related gene in a human mammary carcinoma, Science, 229 (4717), 974-976 (1985))
(2) Semba K, Kamata N, Toyoshima K, Yamamoto T., A v-erbB-related protooncogene, c-erbB-2, is distinct from the c-erbB-1/epidermal growth factor-receptor gene and is amplified in a human salivary gland adenocarcinoma, Proc. Natl. Acad. Sci. USA,82: 6497-6501, 1985.
(3) Coussens L, Yang-Feng TL, Liao YC, et al., Tyrosine kinase receptor with extensive homology to EGF receptor shares chromosomal location with neu oncogene, Science, 230: 1132-1139, 1985.

It is reported that the amplification of HER2 gene and overexpression of protein in 10 to 30% of human patients with breast cancer. This overexpression can be determined by detection of genes (FISH, CISH, PCR, Southern blotting, etc.), detection of mRNA expression (ISH, northern blotting, etc.), detection of protein expression (immunohistochemistry (IHC), western blotting, enzymeimmunoassay (EIA), etc.) (Bilous M., Dowsett M., Hanna W., Isola J., Lebeau A., Moreno A., Penault-Llorca F., Ruschoff J., Tomasic G, can de Vijver M., Current perspectives on HER2 testing: a review of national testing guidelines, Mod. Pathol., 16: 173-182, 2003.).

Using these known methods, the samples determined to overexpress HER2 are judged HER2-positive in the present invention. The following testing methods are known as the methods approved by the FDA in USA and in Japan and Europe, which have been clinically applied.
FISH: DakoCytomation HER2 FISH pharmDx^{™} Kit (DaKo Cytomation Corp.) PathVysion HER2 DNA Probe Kit (Abbott Laboratories)
IHC (immunohistochemistry): HercepTest^{™} (DaKo Cytomation Corp.)
EIA (enzyme-linked immunoassay): HER2/neu ELISA (Oncogene Science Inc.) ErbB-2 EIA "NICHIREI" (Nichirei Corp.)

In the present invention, the negative/positive status of HER2 was assessed using the HercepTest (trademark: HercepTest, DAKO Corporation), in which the expression of HER2 protein is detected by immunohistochemistry (IHC).

The HerceptTest (trademark) is approved by the FDA in USA and the Ministry of Health and Welfare in Japan as a screening kit for breast cancer, where samples are assessed by following the instructions given. The HerceptTest (trademark) can be performed according to the Dako Corporation's manuals. Samples stained by HerceptTest (trademark) are assigned a score with strict adherence to the instructions given; more specifically, when samples demonstrate weak to moderate positive staining of the complete cell membrane in ≧10% of cancer cells, the samples are determined to overexpress HER2 and judged to be HER2-positive in the present invention.

For instance, the images of samples stained with HercepTest are observed, and scored as 0, 1+, 2+ and 3+, which are classified into the following categories.
0: No positive staining of the cell membrane or positive staining of the cell membrane in ≧ 10% of cancer cells (cytoplasmic localization of positive staining is not included in the assessment).
1+: Faint, barely perceptible staining of the cell membrane in ≧10% of cancer cells; the cancer cells are stained only in part of their cell membrane.
2+: Weak to moderate positive staining of the complete cell membrane in ≥10% of cancer cells.
3+: Intense positive staining of the complete cell membrane in ≥10% of cancer cells.

The samples scored equal to or greater than 2+ or 3+ are found to overexpress the HER2 protein (HER2-positive in the present invention) and the samples scored equal to or less than +1 can be defined as no overexpression of the HER2 protein (HER2-negative in the present invention).

### 1.2.2 Hormone-sensitive breast cancer

In breast cancer, 60 to 80% expresses receptors to female hormones. It is thus said that stimulation of the female hormones (estrogen, progesterone, etc.) affects the growth of cancer in breast cancer where the female hormones are expressed.

Currently, it is possible to determine by inspecting the hormone receptors in biopsy specimens of breast cancer or surgical specimens of breast cancer whether breast cancer can be affected by female hormones. The breast cancer which expresses either ER or PgR or both receptors and is susceptible to the effects of female hormones is called "hormone-sensitive cancer" or "hormone-dependent cancer." It is revealed that highly therapeutic effects are obtained in this type of breast cancer.

In premenopausal women whose ovarian functions are active, the ovary is the main source of female hormones. It is known that male hormones secreted from the adrenal cortex are the precursor for female hormone biosynthesis in postmenopausal women.

Anti-hormone agents including anti-estrogen agents, selective aromatase inhibitors, luteinizing hormone release stimulating hormone inhibitors, etc. are expected to be effective for the "hormone-sensitive breast cancer" or "hormone-dependent breast cancer." However, it is known that there are patients who have not expressed the hormone receptors intrinsically or patients who have lost the receptors during drug therapy to become irresponsive to the therapy (inadaptability/resistance), and therapeutic drugs for the hormone receptor-negative cancer, "hormone-insensitive breast cancer" or "hormone-independent breast cancer" have been strongly requested.

The present invention responds to such a request and provides the agent for the prevention or treatment of a breast cancer including an ER-negative and HER2-negative breast cancer, a PgR-negative and HER2-negative breast cancer and an ER-negative, PgR-negative and HER2-negative breast cancer, which comprises the Akt inhibitor.

### 1.2.3. Assessment test for the negative/positive status of ER (estrogen receptors)

ER (estrogen receptor) is a receptor for estrogen, which is one of female hormones. It is reported that the ER gene is expressed in 83.2% of human patients with breast cancer (Harvey, J.M., Clark, G.M., Osborne, C.K., Allred D.C., Estrogen receptor status by immunohistochemistry is superior to the ligand-binding assay for predicting response to adjuvant endocrine therapy in breast cancer: J. Clin. Oncol., 17: 1474-1481, 1999.).

ER has been deposited under Accession Nos. Gene: NM_000125, Protein: NP_000116 (Greene, GL., Gilna, P., Waterfield, M., Baker, A., Hort, Y. and Shine, J. Sequence and expression of human estrogen receptor complementary DNA: Science, 231 (4742), 1150-1154 (1986), etc.). The ER comprises two types of estrogen receptors that are ER1 (also known as ERα) and ER2 (also known as ERβ).

A representative of the clinical tests which are used to detect the expression of estrogen receptors is a method using immunohistochemical staining.

Clone 1D5 (Dako Cytomation Corp.), 6F11 (Ventana Japan), ER88 (Kyowa Medix Inc.) and the like are commercially available as in vitro diagnostic antibodies. These antibodies can be stained using an automated staining device. Clone 1D5 and ER88 can also be stained by manual operation.

Furthermore, test methods which have been approved and clinically applied in USA, Japan and Europe include:
IHC (immunohistochemistry),
ENVISION+ Kit/HRP (DAB)-ER, ID5-PgR, PgR636 (Dako Cytomation),
"Kyowa Stain" ER (M) (Kyowa Medex)
and the like.

In the present invention, the positive/negative status ofER was determined by immunohistochemistry (IHC) using Ventana Confirm EstrogenReceptor (6F11) (registered trademark: Ventana) for detecting the expression ofER protein.

Ventana Confirm (registered trademark) can be performed by following the manuals of Ventana Corp. In the samples stained with Ventana Confirm (registered trademark), the entire tumor is observed under a x4 objective lens of optical microscope, whereby immunoreactivity of ER can be confirmed in the nucleus of tumor cells. The cell which has the nucleus found to be immunoreactive is defined as a positive cell. The rate of the positive cell to the entire tumor is calculated in terms of percentage irrespective of its staining intensity. When the positive cell rate is 10% or greater, the sample can be defined as ER-positive, based on the percentage calculated.

### 1.2.4. Assessment test for the negative/positive status of PgR (progesterone receptors)

PgR (progesterone receptor) is a receptor for progesterone, which is one of female hormones. It is reported that the PgR gene is expressed in 73.0% of human patients with breast cancer (Syed K. Mohsin, Heidi Welss, Thomas Havighurst, Gary M. Clark, Melora Berardo, Le D. Roanh, Ta V To, Qian Zho, Richard R. Love and D. Craig Allred, Progesterone receptor by immunohistochemistry and clinical outcome in breast cancer: a validation study, Modern Pathology, (2004), 17, 1545-1554).

In the PgR-positive breast cancer, female hormones such as progesterone, etc. are involved in the growth of breast cancer cells. Accordingly, the effects of anti-hormone agents including anti-estrogen agents, selective aromatase inhibitors, luteinizing hormone release stimulating hormone inhibitors, etc. can be expected. However, it is known that there are patients who have not expressed the PgR intrinsically or patients who have lost the PgR during drug therapy to become irresponsive to the therapy (inadaptability/resistance), and therapeutic drugs for PgR receptor-negative cancer have been strongly requested.

PgR has been deposited under Accession Nos. Gene: NM_000926.2, Protein: NP_000917 Fernandez, M.D., Carter, GD. and Palmer, T.N. The interaction of canrenone with oestrogen and progesterone receptors in human uterine cytosol: Birth Defects Res. Part A, Clin. Mol. Teratol., 15 (1), 95-101 (1983), Misrahi, M., Atger, M., d'Auriol, L., Loosfelt, H., Meriel, C., Fridlansky, F., Guiochon-Mantel, A., Galibert, F., Milgrom, E., Complete amino acid sequence of the human progesterone receptor deduced from cloned cDNA, Biochem. Biophys. Res. Commun., 143: 740-748, 1987, etc.).

Clone Pgr636 (Dako Cytomation Corp.), 1A6 (Ventana Japan), PR88 (Kyowa Medix Inc.) and the like are commercially available as in vitro diagnostic antibodies used to determine the expression of progesterone receptors. These antibodies can be stained using an automated staining device. Clone Pgr636 and PR88 can also be stained by manual operation.

Furthermore, test methods which have been approved and clinically applied in USA, Japan and Europe include:
IHC (immunohistochemistry),
ENVISION+ Kit/HRP (DAB)-ER, 1D5-PgR, PgR636 (Dako Cytomation), "Kyowa Stain" PgR(M) (Kyowa Medex)
and the like.

In the present invention, the positive/negative status ofER was determined by immunohistochemistry (IHC) using Ventana Confirm EstrogenReceptor (6F11) (registered trademark: Ventana) for detecting the expression of PgR protein.

Ventana Confirm (registered trademark) can be performed by following the manuals of Ventana Corp. In the samples stained with Ventana Confirm (registered trademark), the entire tumor is observed under a x4 objective lens of optical microscope, whereby immunoreactivity of PgR can be confirmed in the nucleus of tumor cells. The cell which has the nucleus found to be immunoreactive is defined as a positive cell. The rate of the positive cell to the entire tumor is calculated in terms of percentage irrespective of its staining intensity. When the positive cell rate is 10% or greater, the sample can be defined as ER-positive, based on the percentage calculated.

### 1.3. Pharmaceutical preparation comprising Akt inhibitor

According to the present invention, the Akt inhibitor can be prepared into a pharmaceutical composition in a conventional manner, which can be used as the agent for the prevention or treatment of breast cancer.

Examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, intraarticular injection, etc. These injectable preparations may be prepared by methods publicly known, e.g., by dissolving, suspending or emulsifying the active ingredients described above in a sterile aqueous medium or oily medium conventionally used for injections. As the aqueous medium for injections, there are, for example, physiological saline, an isotonic solution containing glucose and other auxiliary agents, etc., which may be used in combination with an appropriate solubilizing aid such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing aid such as benzyl benzoate, benzyl alcohol, etc. The injection thus prepared is preferably filled in an appropriate ampoule. The suppository used for rectal administration may be prepared by blending the aforesaid substance with conventional bases for suppositories.

Each composition described above may further contain other active ingredients unless formulation causes any adverse interaction with the active ingredients described above.

A dose of the active ingredient may vary depending upon its effects, target disease, subject to be administered, conditions, route of administration, etc.: in oral administration, the active ingredient is generally administered to an adult (as 60 kg body weight) in a daily dose of about 0.1 to about 100 mg, preferably about 1.0 to about 50 mg and more preferably about 1.0 to about 20 mg. In parenteral administration, a dose of the active ingredient may vary depending upon the target disease, subject to be administered, conditions, route of administration, etc.; in the form of an injectable dosage form, it is advantageous to administer the active ingredient to an adult (as 60 kg body weight) generally in a daily dose of about 0.01 to about 30 mg, preferably about 0.1 to about 20 mg, and more preferably about 0.1 to about 10 mg. For other animal species, the corresponding dose as converted per 60 kg weight can be administered.

The antisense polynucleotide described above can be subjected to pharmaceutical manufacturing processes according to publicly known methods and then administered. The antisense polynucleotide alone can be administered directly, or the antisense polynucleotide can be inserted into an appropriate vector such as retrovirus vector, adenovirus vector, adenovirus-associated virus vector, etc., and then administered orally or parenterally to human or a mammal (e.g., rat, rabbit, sheep, swine, bovine, cat, dog, monkey, etc.) in a conventional manner. The antisense polynucleotide may be administered as it stands, or may also be subjected to pharmaceutical manufacturing processes together with a physiologically acceptable carrier such as a pharmaceutical aid to assist its uptake, which are then administered by gene gun or through a catheter such as a catheter with a hydrogel. Alternatively, the antisense polynucleotide may be prepared into an aerosol, which is topically administered into the trachea as an inhaler. Further for the purposes of improving pharmacokinetics, prolonging a half-life and improving intracellular uptake efficiency, the antisense polynucleotide described above is prepared into pharmaceutical preparations (injectable preparations), alone or together with a carrier such as liposome, etc. and the preparations may be administered intravenously, subcutaneously or intraarticularly, or at the site of cancerous lesion, etc. The aforesaid double-stranded RNA or ribozyme, or the aforesaid variant of the protein used in the present invention which acts dominant negatively against the protein used in the present invention or the polynucleotide encoding the same can be likewise prepared into pharmaceutical preparations as in the antisense polynucleotide described above, which preparations can be provided for administration.

The antibody, aptamer and the like described above can be administered directly as it is or in the form of an appropriate pharmaceutical composition. The pharmaceutical composition used for the administration described above contains the aforesaid antibody or its salt and a pharmacologically acceptable carrier diluent or excipient. Such a composition is provided in the preparation suitable for oral or parenteral administration (e.g., intravenous injection). Preferably, the composition is provided as an inhaler.

### 2. Method of screening the agent for the prevention or treatment of breast cancer

Next, the present invention provides the method of screening the agent for the prevention or treatment of the breast cancer described above, using the Akt inhibitory activity as an indicator, and more specifically, provides the method of screening the agent for the prevention or treatment of the breast cancer which is hormone receptor-negative (ER-negative and/or PgR-negative) and HER2-negative.

A preferred embodiment of the screening method of the present invention is a method which comprises evaluating the Akt phosphorylation inhibitory activity of a test compound and selecting a compound having the Akt inhibitory activity. The compound selected as having the Akt inhibitory activity is a candidate compound for the agent for the prevention or treatment of a hormone receptor-negative (ER-negative and/or PgR-negative) and HER2-negative breast cancer.

Examples of the test compound include peptides, proteins, antibodies, non-peptide compounds, synthetic compounds, fermentation products, cell extracts, plant extracts, animal tissue extracts, blood plasma, and the like. These compounds may be novel or publicly known compounds. The test compound may form a salt, and the salts of the compound include physiologically acceptable metal salts, ammonium salts, salts with organic bases, salts with inorganic acids, salts with organic acids, salts with basic or acidic amino acids, etc. Preferred examples of the metal salts include alkali metal salts such as sodium salts, potassium salts, etc.; alkaline earth meal salts such as calcium salts, magnesium salts, barium salts, etc.; aluminum salts, etc. Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Preferred examples of the salts with inorganic acids include salts with hydrochloric acid, hydrobromic acid, nitric acid, sulfuric acid, phosphoric acid, etc. Preferred examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferred examples of the salts with basic amino acids include salts with arginine, lysine, ornithine, etc., and preferred examples of the salts with acidic amino acids include salts with aspartic acid, glutamic acid, etc.

More specifically, the present invention provides, for example, (1) the method of screening an agent for the prevention or treatment of a hormone receptor-negative (ER-negative and/or PgR-negative) and HER2-negative breast cancer, which comprises comparing the level of phosphorylation of Akt in a breast cancer cell, (i) when a test compound is brought in contact with the cell and (ii) when a test compound is not brought in contact with the cell.

According to this method, first, a test compound is contacted with a breast cancer cell that is hormone receptor-negative (ER-negative and/or PgR-negative) and HER2-negative. The "cells" used are not limited to a particular origin but human breast cancer cells are preferred. The breast cancer cells which are hormone receptor-negative and HER2-negative can also be produced by general genetic engineering techniques through knockout of a gene encoding ER and/or PgR and HER2 to inhibit its expression, or the like.

Next, the Akt phosphorylation activity is determined. Specifically, the cells described above are cultured to assay their levels of phosphorylated Akt protein in the cases of (i) and (ii). The level of phosphorylated Akt protein can be assayed by publicly known methods, for example, western blotting or ELISA assays using a phosphorylation antibody capable of specifically reacting with the phosphorylated Akt. The Akt phosphorylation can also be determined by assaying the transcription factor located downstream of the protein involved in Akt signal transduction, for example, for its ability of transcriptional activation by isolating a promoter region from the target gene encoding the transcription factor in a conventional manner, inserting a labeled gene (e.g., a light-emitting, fluorescent or chromogenic gene such as luciferase, GFP, galactosidase, etc.) downstream of the promoter region and assaying the activity of the labeled gene. The compounds as given above are likewise used as the test compound.

The enzyme inhibitory activity of Akt can also be determined using HTScan^{™} Akt1 Kinase Assay Kit (Cell Signaling Technology, Catalog No. 7501), HTScan^{™} Akt2 Kinase Assay Kit (Cell Signaling Technology, Catalog No. 7504), HTScan^{™} Akt3 Kinase Assay Kit (Cell Signaling Technology, Catalog No. 7507), etc.

The intracellular Akt inhibitory activity can also be determined by the methods described in the literatures below. Kozikowski, A. P., Sun, H., Brognard, J., Dennis, P. A., Novel PI Analogues Selectively Block Activation of the pro-survival Serine/Threonine Kinase Akt, J. Am. Chem. Soc., (Communication); 2003; 125(5); 1144-1145

Next, the compound that suppresses (reduces) the phosphorylation of Akt when compared to the case where the test compound is not contacted (control) is selected. A test compound that suppresses (reduces) the phosphorylation of Akt in the case of (i) described above, for example, by at least about 20%, preferably at least 30%, more preferably at least about 50% when compared to the case of (ii) described above can be selected as the compound that suppresses (reduces) the phosphorylation of Akt. The compound thus selected is such a compound that inhibits the phosphorylation of Akt and can be a candidate compound for the agent for preventing or treating the hormone receptor-negative and HER2-negative breast cancer.

In the specification and the sequence listings, where nucleotides, amino acids, etc. are denoted by their abbreviations, they are based on conventional codes in accordance with the IUPAC-IUB Commission on Biochemical Nomenclature or by the common codes in the art, examples of which are shown below. For amino acids that may have the optical isomer, L form is presented unless otherwise indicated.

### EXAMPLES

Next, the present invention will be described in detail by referring to EXAMPLES but is not deemed to be limited thereto.

A portion (100 mg or more) of breast cancer tissues surgically removed was frozen in liquid nitrogen immediately after resection and stored at -80°C. The frozen breast cancer tissues were immediately fixed in 10% neutral formalin for 24-48 hours and embedded in paraffin. The formalin-fixed samples were used for immunohistochemical analysis.

The formalin-fixed samples were sliced in a thickness of 4-6 µm and spread onto a slide to prepare the sample slides. Using the sample slides, overexpression of the HER2 protein was analyzed using HercepTest^{™} (Dako Cytomation). Furthermore, the estrogen receptor (Clone 6F 11) and the progesterone receptor (Clone 1A6) were analyzed using CONFIRM^{™} (Ventana Japan). The staining method and assessment criteria were both basically in accordance with the manufacturers' protocols.

Specific steps for the immunohistochemical staining of HER2 are as follows.

### (a) Deparaffinization

### (b) Antigen retrieval treatment

(1) Fill a staining dish with an antigen retrieval solution and immerse the staining dish in a water bath previously to warm the staining dish.
(2) Put the sample tissue slides into the water bath.
(3) Confirm that the temperature of the antigen retrieval solution temporarily decreased has risen to 95-99°C and then heat for 40 minutes.
(4) Maintain the temperature at 95-99°C and close the lid of the staining dish to prevent water evaporation.
(5) Remove the staining dish from the water bath, take the lid off and leave to stand: 20 minutes at ambient temperature.
(6) Rinse under running water: 3 minutes.
(7) Immerse in wash buffer: 5 minutes.

### (c) Blocking of endogenous peroxidase

(1) Add 2 drops of blocking reagent: reaction at ambient temperature for 5 minutes.
(2) Rinse under running water: 3 minutes.
(3) Immerse in wash buffer: 5 minutes.

### (d) Reaction of primary antibody

(1) Carefully wipe away excess water on the slide.
(2) Add 2 drops of a primary antibody: react at ambient temperature for 30 minutes.
(3) Rinse in wash buffer.
(4) Immerse in wash buffer: 5 minutes x 2

### (e) Reaction of polymer reagent

(1) Carefully wipe away excess water on the slide.
(2) Add 2 drops of polymer reagent: react at ambient temperature for 30 minutes.
(3) Rinse in wash buffer.
(4) Immerse in wash buffer: 5 minutes x 2

### (f) Color development with substrate solution

(1) Carefully wipe away excess water on the slide.
(2) Dropwise add 100 µl of the substrate solution prepared.
(3) React at ambient temperature for 10 minutes..
(4) Rinse with distilled water.
(5) Immerse in distilled water.

### (g) Counterstaining

### (h) Sealing

ER- and PgR-immunohistochemical staining was performed using an automated immunostaining device Benchmark XT (trademark; Ventana Japan), which is fully automated from the deparaffinization to the color development. The protocols are outlined below.
(a) Deparaffinization
(b) Antigen retrieval treatment: heat in standard CC1 buffer at 100°C for 60 minutes.
(c) Reaction with primary antibody: react at room temperature for 32 minutes using a primary antibody (ER: 6F11, PgR16).
(d) Reaction with secondary antibody: reaction using LSAB kit.
(e) Color development

Scoring was performed by the criteria shown below to assess the positive/negative status of HER2 in each sample. HER2-immunohistochemical staining scores (HER2 IHC)
0: No positive staining of the cell membrane or positive staining of the cell membrane in < 10% of cancer cells (cytoplasmic localization of positive staining is not included in the assessment).
1+: Faint, barely perceptible staining of the cell membrane in ≥10% of cancer cells; the cancer cells are stained only in part of their cell membrane.
2+: Weak to moderate positive staining of the complete cell membrane in ≥ 10% of cancer cells.
3+: Intense positive staining of the complete cell membrane in ≥ 10% of cancer cells.

Scores 0 and 1+ were judged negative and scores 2+ and 3+ were judged positive.

Scoring was performed by the criteria shown below to assess the positive/negative status of ER and PgR in each sample.
0: negative (10% ≧)
1+: 10% ≦ positive cell count <50%
2+: 50% ≦ in positive cell count

Scores 0 was judged negative and scores 1+, 2+ and 3+ were judged positive.

Using the above-described portion of breast cancer tissues surgically removed, biochemical analysis (western blotting) was performed. After the frozen and stored tissue section was physically disrupted, RIPA buffer (50mM Tris hydrochloride: pH 8.0, 150 mM sodium chloride, 0.02% sodium azide, 0.1% SDS, 1% Nonidet P-40, 0.5% sodium deoxycholate) was added thereto. After homogenization, the insoluble fraction was removed by centrifugation to give the tissue extract. Using a DC protein assay kit (manufactured by BioRad, Code No. 500-0120), the protein level in the tissue extract was quantified. An equivolume of 2-fold concentrated SDS sample buffer was mixed with 10 µg of the protein and the mixture was heat-treated at 95°C for 5 minutes to give the tissue lysate. The western blotting analysis was performed as follows. More specifically, this tissue lysate was electrophoresed by SDS-PAGE electrophoresis (gel concentration: 7.5 - 15%) (Analytical Biochemistry, 166, 368-379, 1987) followed by transfer onto a nitrocellulose membrane (BioRad).

Thereafter, the objective proteins on the nitrocellulose membrane were detected using antibodies specific to the respective proteins and antibodies specific to the respective phosphorylations.

Akt-specific antibody (manufactured by Cell Signaling Technology, Code No. 9272), anti-phosphorylated Akt antibody (manufactured by Cell Signaling Technology, Code No. 9271), MAPK-specific antibody (manufactured by Cell Signaling Technology, Code No. 9102), anti-phosphorylated MAPK antibody (manufactured by Cell Signaling Technology, Code No. 9101), HER2-specific antibody (manufactured by Cell Signaling Technology, Code No. 2242) and phosphorylated HER2 antibody (manufactured by Cell Signaling Technology, Code No. 2244) were used as the primary antibodies.

Using HRP-bound anti-rabbit antibody (manufactured by Cell Signaling Technology, Code No. 7074) as the secondary antibody, the ECL system (Amersham) was used to detect the antibodies. LAS-1000 Plus (Fuji Photo Film) was used for visualization and image analysis to determine the respective protein levels and phosphorylated protein levels, and their weight ratios were calculated.

Forty four samples collected from the patients with breast cancer were classified by tissue type and the expression patterns of, ER, PgR and HER2, which matched general incidence and no particular deviation was observed.
a) Tissue type

| | |
|---|---|
| Noninfiltrating cancer | 5/44 (11.4%) |
| Infiltrating cancer | 39/44 (88.6%) |

(b)

| | |
|---|---|
| ER-positive | 33/44 (75.0%), |
| ER-negative | 11/44 (25.0%) |
| PgR-positive | 29/44 (65.9%) |
| PgR-negative | 15/44 (34.1%) |
| HER2-positive | 14/44 (31.8%), |
| HER2-negative | 30/44 (68.2%) |

(c)

| | |
|---|---|
| A-ER group: ER-positive and HER2-negative | 21/44 (47.7%) |
| B-ER group: ER-positive and HER2-positive | 12/44 (27.3%) |
| C-ER group: ER-negative and HER2-positive | 3/44 (6.8%) |
| D-ER group: ER-negative and HER2-negative | 8/44 (18.2%) |
| A-PgR group: PgR-positive and HER2-negative | 20/44 (45.4%) |
| B-PgR group: PgR-positive and HER2-positive | 9/44 (20.5%) |
| C-PgR group: PgR-negative and HER2-positive | 6/44 (14.6%) |
| D-PgR group: PgR-negative and HER2-negative | 9/44 (20.5%) |

(d)

| | |
|---|---|
| Group (i): ER-positive, PgR-positive and HER2-negative | 20/44 (45.5%) |
| Group (ii): ER-positive, PgR-negative and HER2-negative | 1/44 (2.3%) |
| Group (iii): ER-positive, PgR-positive and HER2-positive | 9/44 (20.5%) |
| Group (iv): ER-positive, PgR-negative and HER2-positive | 3/44 (6.8%) |
| Group (v): ER-negative, PgR-negative and HER2-positive | 3/44 (6.8%) |
| Group (vi): ER-negative, PgR-negative and HER2-negative | 8/44 (18.2%) |

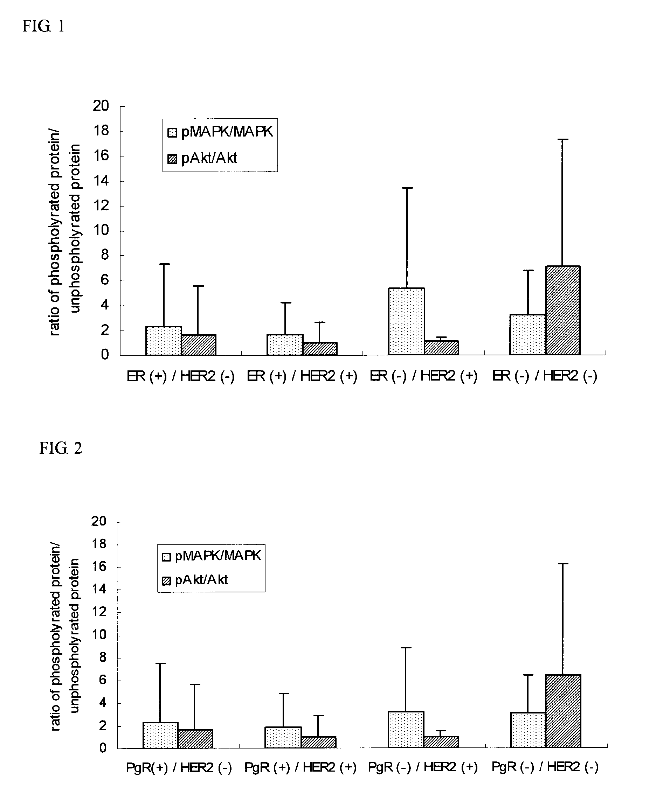

The mean values for ratios of the protein levels to the phosphorylated protein levels of HER2, Akt and MAPK in each group of the A-ER, B-ER, C-ER and D-ER groups classified by dividing samples according to the category (c) are shown in FIG 1.

In the C-ER and D-ER groups which are ER-negative, the phosphorylation of MAPK was clearly upregulated as compared to the A-ER and B-ER groups which are ER-positive. In other words, it is suggested that the MAPK inhibitor would be effective as the agent for the prevention or treatment of an estrogen receptor-negative breast cancer.

Further in the D-ER group, it became clear that the phosphorylation of Akt was significantly upregulated as compared to the A-ER, B-ER and C-ER groups. In other words, it is revealed that the Akt inhibitor is effective as the agent for the prevention or treatment of an estrogen receptor-negative and HER2-negative breast cancer.

The mean values in the ratios of the protein levels and phosphorylated protein levels of HER2, Akt and MAPK in each group of the A-PgR, B-PgR, C-PgR and D-PgR groups classified by the category (c) are shown in FIG 2.

In the C-PgR and D-PgR groups which are PgR-negative, it became clear that the phosphorylation of MAPK was upregulated as compared to the A-PgR and B-PgR groups which are PgR-positive. In other words, it is suggested that the MAPK inhibitor would be effective as the agent for the prevention or treatment of a progesterone receptor-negative breast cancer.

Also in the D-PgR group, it became clear that the phosphorylation of Akt was upregulated as compared to the A-PgR and B-PgR groups. In other words, it is revealed that the Akt inhibitor is effective as the agent for the prevention or treatment of a progesterone receptor-negative and HER2-negative breast cancer.

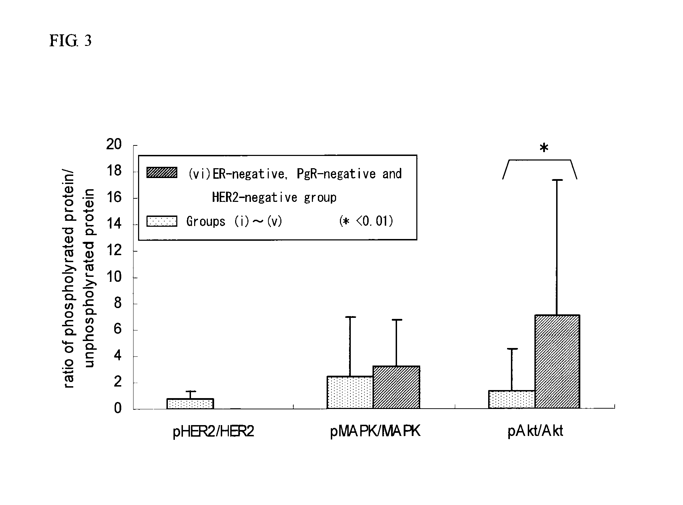

The mean values in the ratios of the protein levels and phosphorylated protein levels of HER2, Akt and MAPK in group (vi) (ER-negative, PgR-negative and HER2-negative group) and the other groups (groups (i) to (v)) classified by the category (d) were studied. The results of this study are shown in FIG. 3.

As shown in FIG. 3, it became clear that the Akt inhibitor in the group (vi) was significantly upregulated as compared to the other groups (i) to (v). In other words, it is revealed that the Akt inhibitor is effective as the agent for the prevention or treatment of an estrogen receptor-negative, progesterone receptor-negative and HER2-negative breast cancer.

### INDUSTRIAL APPLICABILITY

According to the present invention, there can be provided the agent for the prevention or treatment of (1) an estrogen receptor-negative and HER2-negative breast cancer, (2) a progesterone receptor-negative and HER2-negative breast cancer, (3) an estrogen receptor-negative, progesterone receptor-negative and HER2-negative breast cancer, (4) an estrogen receptor-negative, progesterone receptor-positive and HER2-negative breast cancer, (5) an estrogen receptor-positive, progesterone receptor-negative and HER2-negative breast cancer, and so on.

Further according to the present invention, there can be provided a method of screening the agent for the prevention or treatment of the breast cancer described above.

## Claims

1. An agent for the prevention or treatment of an estrogen receptor-negative and HER2-negative breast cancer comprising an Akt inhibitor.

2. An agent for the prevention or treatment of an estrogen receptor-negative, progesterone receptor-negative and HER2-negative breast cancer comprising an Akt inhibitor.

3. The agent for the prevention or treatment of the breast cancer according to claim 1 or 2, wherein Akt is at least one selected from Akt1, Akt2 and Akt3.

4. The agent for the prevention or treatment of the breast cancer according to claim 1 or 2, wherein the Akt inhibitor is at least one selected from the group consisting of (a) a low molecular weight compound or high molecular weight compound which inhibits the phosphorylation of Akt, (b) a low molecular weight compound or high molecular weight compound which inhibits the expression of Akt, (c) an antibody which inhibits the phosphorylation of Akt, (d) an antibody which inhibits the expression of Akt, (e) a siRNA or shRNA against a polynucleotide encoding Akt, (f) an antisense polynucleotide comprising a nucleotide sequence complementary or substantially complementary to the nucleotide sequence of a polynucleotide encoding Akt, or comprising a part of said nucleotide sequence, (g) a ribozyme directed to a polynucleotide encoding Akt, (h) a mutant of Akt which dominant-negatively acts on Akt or a polynucleotide encoding said mutant, and (i) an aptamer against Akt.

5. A method of screening an agent for the prevention or treatment of an estrogen receptor-negative and HER2-negative breast cancer, which comprises using an Akt inhibitory activity as an indicator.

6. A method of screening an agent for the prevention or treatment of an estrogen receptor-negative, progesterone receptor-negative and HER2-negative breast cancer, which comprises using an Akt inhibitory activity as an indicator.
